# EUROPÄISCHE PATENTSCHRIFT

(11) **EP 0 818 436 B1**
(45) Veröffentlichungstag und Bekanntmachung des Hinweises auf die Patenterteilung: **20.03.2002**
(21) Anmeldenummer: 97110358.5
(22) Anmeldetag: 25.06.1997
(51) Int. Cl.: C07C 209/42, C07C 245/08

(54) **Verfahren zur Herstellung von 4-Hydroxyanilinen**
Process for the preparation of 4-hydroxyanilines
Procédé de préparation de 4-hydroxyanilines

(30) Priorität: 08.07.1996 DE 19627424
(43) Veröffentlichungstag der Anmeldung: 14.01.1998
(73) Patentinhaber: BAYER AG, 51368 Leverkusen (DE)
(72) Erfinder: Hupperts, Achim, Dr., 40217 Düsseldorf (DE); Steinbeck, Karl, Dr., 51399 Burscheid (DE); Stelzer, Uwe, Dr., 51399 Burscheid (DE); Lantzsch, Reinhard, Dr., 42115 Wuppertal (DE); Seifert, Hermann, Dr., 51467 Bergisch Gladbach (DE)

(56) Entgegenhaltungen:
- DE-A- 4 040 853
- FR-A- 2 487 832
- US-A- 2 362 508
- CHEMICAL ABSTRACTS, vol. 125, no. 15, 7.Oktober 1996 Columbus, Ohio, US; abstract no. 195105t, STELIAN RADU ET AL.: "Synthesis of some 4-(phenylazo)phenoxyacetic acids with active physicological properties by condensation of sodium 4-(phenylazo) phenoxides with monochloroacetic acid." Seite 1155; Spalte 1; XP002044025 & REV. CHIM. , Bd. 47, Nr. 5, 1996, BUCHAREST, Seiten 411-415,
- CHEMICAL ABSTRACTS, vol. 124, no. 5, 29.Januar 1996 Columbus, Ohio, US; abstract no. 49140p, MURALIKRISHNA CHIVUKULA ET AL.: "Phenolic azo dye oxidation by laccase from pyricularia oryzae" Seite 531; Spalte 2; XP002044026 & APPL. ENVIRON. MICROBIOL., Bd. 61, Nr. 12, 1995, Seiten 4374-4377,
- JEFFREY L. JURLINA ET AL.: "Redox Chemistry of the 9-Anilinocridine Class of Antitumor Agents" JOURNAL OF MEDICINAL CHEMISTRY, Bd. 30, Nr. 3, März 1987, WASHINGTON US, Seiten 473-480, XP002044023
- P. BOSSHARD ET AL.: "Weitere Reaktionen an Acylchinonen und verwandten Verbindungen" HELVETICA CHIMICA ACTA, Bd. 47, Nr. 3, 20.April 1964, BASEL CH, Seiten 769-784, XP002044024
- MARCH J.: 'Advanced Organic Chemistry', 1985, WILEY, NEW YORK, 3. AUFLAGE * Seite 471 *
- WHITMORE W.F. ET AL.: 'The Quantitative Hydrogenation of Substituted Azo Compounds with Raney Nickel at Normal Temperature and Pressure' JOURNAL OF THE AMERICAN CHEMICAL SOCIETY Bd. 62, 1940, EASTON, US, Seiten 1687 - 1693
- MORRISON R.T., BOYD R.N.: 'Organic Chemistry', 1980, ALLYN AND BACON, BOSTON, MASSACHUSETTS. 3. AUFLAGE * Seite 342 - Seite 344 *

## Beschreibung

Die Anmeldung betrifft die Herstellung von 2,3-disubstituierten 4-Hydroxyanilinen durch Umsetzung von 2,3-disubstituierten Phenolen mit Diazoniumsalzen und anschließender Reduktion.

2,3-disubstituierte 4-Hydroxyaniline sind wichtige Zwischenprodukte zur Herstellung von Pflanzenschutzmitteln und werden in der DE-4 040 853 beschrieben.

Die Reduktion von 9-Anilincaridinen wird in Penny et. al., J. Med Chem. 1987, 30, 473-480 beschrieben.

Es wurde nun ein neues Verfahren zur Herstellung von 2,3-disubstituierten 4-Hydroxyanilinen gefunden, das sich durch sehr hohe und reproduzierbare Ausbeuten, einfache Reinigungs- und Aufarbeitungsstufen sowie schonende Reaktionsbedingungen auszeichnet und bei dem keine Nebenprodukte und inaktive Isomere entstehen.

Gegenstand der Anmeldung ist daher ein Verfahren zur Herstellung von Verbindungen der Formel (I) 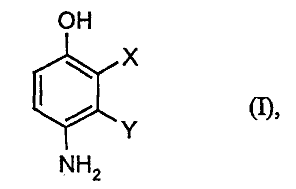 in welcher
- X und Y: unabhängig voneinander jeweils für Halogen und CN stehen
durch Umsetzung von
a) Phenolen der Formel (II) 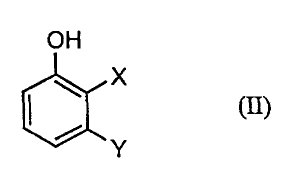 in welcher
   - X und Y: die oben angegebenen Bedeutungen haben,
   mit Diazoniumsalzen der Formel (III) 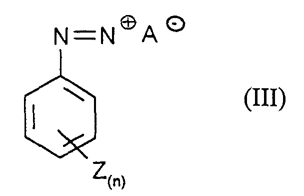 in welcher
   - n: für eine Zahl 0 bis 5,
   - Z: für Halogen, Alkyl, NO₂, COOH, CN und SO₃H steht, wobei Z bei n ≥2 gegebenenfalls unterschiedliche Bedeutungen hat
   - A⁻: für ein Gegenion steht,
   und
b) anschließender Reduktion des nach Verfahrensschritt a) erhältlichen Diazens der Formel (IV) 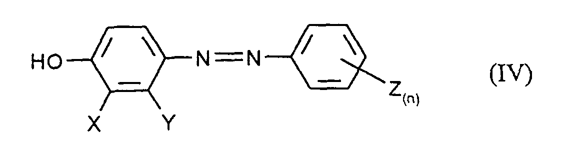 zu der Zielverbindung.

Die Durchführung des Verfahrensschrittes a) erfolgt vorzugsweise dadurch, daß eine Lösung der Diazoniumsalze der Formel (III) zu einer wäßrigen, Lösung der Phenole der Formel (II) gegeben wird. Die Umsetzung erfolgt bei Temperaturen von 0 bis 50°C, vorzugsweise 0 bis 20°C. Der pH-Wert der Lösung liegt vorzugsweise höher als der pKs-Wert des Phenols der Formel (II).

Die Mischung wird anschließend vorzugsweise mit Mineralsäuren auf einen pH-Wert ≤7 eingestellt und das Diazen der Formel (IV) durch Extraktion mit einem organischen, nicht mit Wasser mischbaren Lösungsmittel wie z.B. Essigester oder Butanol extrahiert.

Die Durchführung des Verfahrensschrittes b) erfolgt vorzugsweise dadurch, dass das gemäß Verfahrensschritt a) erhältliche Diazen der Formel (IV) in Gegenwart inerter Lösungsmittel wie vorzugsweise Wasser, Alkoholen wie Methanol, Ethanol oder Butanol, Kohlenwasserstoffe wie z.B. Toluol oder Dioxan, Pyridin, Dimethylformamid oder Gemischen dieser Lösungsmittel hydriert wird.

Bevorzugt wird die gemäß Verfahren a) erhaltene Reaktionslösung direkt ohne weitere Reinigung gemäß Verfahren b) umgesetzt.

Die Verwendung von 2,3-Dichlor-4(4'-nitrophenylazo)-phenol ist in Radu et. al., Revista de Chimie 1996, 47(5), 412-415 beschrieben.

Die Hydrierung erfolgt vorzugsweise mittels Wasserstoff oder Hydrazinen in Gegenwart eines Katalysators und gegebenenfalls in Gegenwart von Hilfsmitteln oder mittels Metallen oder Metallsalzen in Gegenwart von Säuren oder mit Natriumdithionit bzw. Jodwasserstoffsäure. Gegebenenfalls ist die Reaktionslösung vor der Hydrierung mit Mineralsäuren zu neutralisieren (pH~7).

Bevorzugte Ausführungsformen für die Hydrierung und insbesondere Katalysatoren und Hilfsmittel werden in der DE-4 428 535 beschrieben. Bevorzugt wird ein Verfahren zur katalytischen Hydrierung in Gegenwart von Katalysatoren, das dadurch gekennzeichnet ist, dass man die Hydrierung in flüssiger Phase und in Gegenwart eines Katalysators enthaltend wenigstens ein Metall oder eine Metallverbindung aus der Gruppe Nickel, Kobalt und die Edelmetalle und in Gegenwart mindestens einer Schwefelverbindung der Formel (V).

R¹-S(O)ₙ-R² (V),

in der
- R¹ und R²: unabhängig voneinander jeweils geradkettiges oder verzweigtes C₁-C₁₂-Alkyl, Hydroxy-C₁-C₁₂-alkyl, Carboxy-C₁-C₁₂-alkyl oder Phenyl bedeuten,
- R¹: zusätzlich Wasserstoff oder CO-C₁-C₁₂-Alkyl bedeuten kann
- R¹ und R²: gemeinsam auch -CH=CH-CH=CH-, -(CH₂)₄-, -(CH₂)₅- oder -(CH₂)₂-X-(CH₂)₂ mit
- X: = Sauerstoff oder Schwefel bedeuten können und
- n: für Null oder 1 steht, durchführt.

Bevorzugte Schwefelverbindungen der Formel (V) sind solche, bei denen R¹ und R² gleich sind und Hydroxy-C₁-C₆-alkyl bedeuten. Ganz besonders bevorzugt ist Bis-(2-Hydroxyethyl)-sulfid.

Als Edelmetalle für den Katalysator kommen z.B. Ruthenium, Rhodium, Palladium, Osmium, Iridium und Platin in Frage. Bevorzugt ist Palladium. Nickel; Cobalt, Edelmetalle oder deren Verbindungen können gegebenenfalls auf einem Träger vorliegen. ein bevorzugtes Trägermaterial ist Kohle.

Die Menge des Katalysators ist nicht kritisch und kann in weiteren Grenzen variiert werden. Beispielsweise kann man 0,02 bis 3 Gew.-%, bevorzugt 0,1 bis 1 Gew.-% Edelmetall-Katalysator, bezogen auf die Verbindung der Formel (IV) einsetzen.

Das Gewichtsverhältnis der eingesetzten Schwefelverbindungen der Formel (V) zu Katalysator kann z.B. 0,001 bis 0,125:1 betragen. Bevorzugt liegt es bei 0,0025 bis 0,025:1, ganz besonders bevorzugt bei 0,005 bis 0,0125:1.

Die Schwefelverbindungen der Formel (V) können als einzelne Verbindungen oder als Mischung verschiedener Einzelverbindungen eingesetzt werden. Vorteilhafterweise werden die Schwefelverbindungen in Form von Lösungen eingesetzt. Beispielsweise können sie in 0,01 bis 1 gew.-%iger wäßriger oder toluolischer Lösung zum Reaktionsgemisch gegeben werden. Man kann die Schwefelverbindungen auch vor der Reaktion dem Katalysator zumischen.

Besonders bevorzugt wird mittels Wasserstoff oder Hydrazinhydrat in Gegenwart von Raney-Nickel und Bis(2-Hydroxyethyl)sulfid oder mittels Eisen- oder Zinkpulver in Gegenwart von Salzsäure hydriert.

Die Reaktionstemperaturen betragen dabei vorzugsweise 0 bis 150°C, insbesondere 10 bis 50°C. Die Reaktion wird vorzugsweise bei Drücken von 1 bis 100 bar, insbesondere 1 bis 20 bar durchgeführt.

Die Aufarbeitung erfolgt nach allgemein üblichen Methoden, vorzugsweise durch Abdestillieren der Lösungsmittel und des gegebenenfalls noch vorhandenen Anilins, Einstellen eines geeigneten pH-Wertes durch Zugabe von Mineralsäuren bzw. Laugen (pH = Isoelektrischer Punkt des Hydroxyanilins) und Reinigung des Rohproduktes durch Waschen oder Umkristallisieren mit einem inerten Lösungsmittel wie z.B. Wasser oder Toluol.

Das gegebenenfalls zurückgewonnene Anilin kann in den Prozess zurückgeführt werden.

Die Menge an eingesetzten Diazoniumsalz ist 1 bis 1,5 mol, bezogen auf 1 mol Phenolverbindung. Wasserstoff wird im allgemeinen in doppelt molarer Menge bezogen auf die Menge an Diazenverbindung eingesetzt, ein Überschuss kann verwendet werden. Metall und Mineralsäuremenge werden ebenfalls in mindestens doppelt molarer Menge bezogen auf die Menge Diazenverbindung bzw. Metall eingesetzt.

Bevorzugt werden Verbindungen der Formel (II) eingesetzt in denen
- X und Y: unabhängig voneinander für Chlor, Brom, Fluor und CN stehen.

Insbesondere werden Verbindungen der Formel (II) eingesetzt in denen
- X: für Chlor und
- Y: für Chlor steht.

Bevorzugt werden Verbindungen der Formel (III) eingesetzt, in denen
- n: für eine Zahl 0, 1, 2 oder 3 und
- Z: für Chlor, Fluor, Brom, NO₂, COOH, CN, SO₃H, Methyl und Ethyl steht und wobei Z bei n ≥2 gegebenenfalls unterschiedliche Bedeutungen hat.

Insbesondere werden Verbindungen der Formel (III) eingesetzt, in denen
- n: für die Zahl 0 steht.

Die Verbindungen der Formel (III) werden nach allgemein üblichem Verfahren erhalten z.B. in dem man 1 mol Anilin der Formel (IIIa) 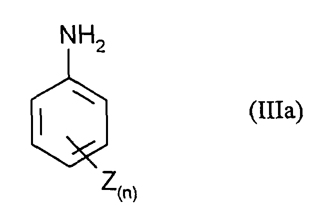 in denen n und Z die oben angegebenen Bedeutungen haben
mit 1 bis 5 mol, vorzugsweise 1,5 bis 2 mol, Nitrit-Verbindungen, wie beispielhaft und vorzugsweise NaNO₂, in verdünnten Mineralsäuren wie z.B. 15 % Salzsäure bei 0 bis 10°C umsetzt.

Mit Hilfe des erfindungsgemäßen Verfahrens werden 2,3-disubstituierte 4-Hydroxyaniline in guter Ausbeute mit hoher Selektivität und Reinheit erhalten. Dabei ist es überaus überraschend, daß in dem Verfahrensschritt a), bei dem gegebenen Substitutionsmuster, eine Reaktion ausschließlich an der zur OH-Gruppe para-stehenden Position stattfindet.

Ebenfalls Gegenstand der Anmeldung sind die nach Verfahren a) erhältliche Verbindung der Formel (IV) in denen X, Y, Z und n die oben angegebenen Bedeutungen haben.

Die Erfindung wird durch die nachfolgenden Beispiele erläutert. Die Erfindung ist nicht auf die Beispiele beschränkt.

### Beispiel 1

In einem 100 ml Zweihalskolben mit Innenthermometer und Magnetrührer wird zu 50 ml wäßriger HCl (15 %) und 9,8 g (0,105 mol) Anilin bei 0 bis 5°C eine Lösung von 7,6 g (0,11 mol) NaNO₂ in 12 ml H₂O zugetropft. Nach 10 Minuten wird diese Diazoniumsalzlösung bei 5 bis 10°C zu einer Lösung von 16,3 g (0,1 mol) 2,3-Dichlorphenol und 20 g (0,5 mol) NaOH in 200 ml H₂O zugegeben. Man läßt das Gemisch auf Raumtemperatur erwärmen, rührt 4 Stunden nach, neutralisiert mit wäßriger HCl und extrahiert 3 x mit je 100 ml Ethylacetat. Die vereinigten organischen Phasen werden mit NaCl-Lösung gewaschen, mit Na₂SO₄ getrocknet und das Lösungsmittel wird im Rotationsverdampfer abdestilliert. Man erhält 31,8 g (2,3-Dichlor-4-hydroxyphenyl)phenyldiazen.

Durch Umkristallisieren aus 200 ml Toluol und 10 ml Methanol können 25,8 g (96,6 % der Theorie) des reinen Diazens gewonnen werden (Schmelzpunkt: 277-279°C).

### Beispiel 2

In einem 500 ml Dreihalskolben mit Innenthermometer, Rührer und Gaseinleitung werden 31,8 g (0,1 mol) nichtgereinigtes, wie aus Beispiel 1 erhaltenes, (2,3-Dichlor-4-hydroxyphenyl)phenyldiazen in 350 ml Methanol unter leichtem Erwärmen gelöst. Der Kolben wird argoniert und anschließend werden 5 g Raney-Nickel und 25 µl Bis(2-Hydroxyethyl)sulfid zugegeben. Bei Raumtemperatur werden dann in 2,5 Stunden 4 640 ml Wasserstoff (0,2 mol) mit ca. 1 bar aufgedrückt. Der Ansatz wird mit etwas Aktivkohle versetzt, weitere 15 Minuten gerührt und über ein Kieselgelpolster filtriert. Nach Abdestillieren des Lösungsmittels erhält man 28,3 g Rohproduktes. Durch Erhitzen mit 100 ml Toluol kann nach Absaugen und Trocknen des Feststoffes 17,0 g (95,5 % der Theorie) reines 4-Hydrodxy-2,3-dichloranilin isoliert werden (Schmelzpunkt: 148-150°C).

### Beispiel 3

In einem 50 ml Zweihalskolben mit Innenthermometer und Rührer werden 3,18 g (0,01 mol) nichtgereinigtes, wie aus Beispiel 1 erhaltenes, (2,3-Dichlor-4-hydroxyphenyl)phenyldiazen in 20 ml Methanol unter leichtem Erwärmen gelöst. Man gibt 1,12 g (0,02 mol) Eisenpulver hinzu und läßt bei Raumtemperatur langsam 3,5 ml (0,04 mol) konzentrierte wäßrige HCI zutropfen. Die Suspension wird 30 Minuten bei Raumtemperatur gerührt, dann mit NaOH neutralisiert und mit 3 x 10 ml Ethylacetat extrahiert. Die vereinigten organischen Phasen werden mit Na₂SO₄ getrocknet und das Lösungsmittel wird im Rotationsverdampfer abdestilliert um 2,01 g Rohprodukt zu erhalten. Durch Erhitzen mit 10 ml Toluol kann nach Absaugen und Trocknen des Feststoffes 1,62 g (91,0 % der Theorie) reines 4-Hydroxy-2,3-dichloranilin isoliert werden (Schmelzpunkt: 148-150°C).

### Beispiel 4

In einem 100 ml Zweihalskolben mit Innenthermometer und Magnetrührer wird zu 50 ml wäßriger HCl (15 %) und 17,0 g (0,105 mol) 2,3-Dichloranilin bei 0 bis 5°C eine Lösung von 7,6 g (0,11 mol NaNO₂ in 12 ml H₂O zugetropft. Nach 10 Minuten wird diese Diazoniumsalzsuspension bei 5 bis 10°C zu einer Lösung von 16,3 g (0,1 mol) 2,3-Dichlorphenol und 20 g (0,5 mol) NaOH in 200 ml H₂O zugegeben. Man läßt das Gemisch auf Raumtemperatur erwärmen, rührt 4 Stunden nach, neutralisiert mit wäßriger HCl und extrahiert 3 x mit je 200 ml Ethylacetat. Die vereinigten organischen Phasen werden über Kieselgur filtriert, mit NaCl-Lösung gewaschen, mit Na₂SO₄ getrocknet und das Lösungsmittel wird im Rotationsverdampfer abdestilliert. Man erhält 29,5 g Diazen. Nach Umkristallisieren aus 200 ml Toluol werden 28,9 g (86 % der Theorie) reines (2,3-Dichlor-4-hydroxyphenyl)-2,3-dichlorphenyldiazen erhalten (Schmelzpunkt 197-199°C).

### Beispiel 5

In einem 100 ml Zweihalskolben mit Innenthermometer und Magnetrührer wird zu 50 ml wäßriger HCl (15 %) und 18,1 g (0,105 mol) Sulfanilsäure bei 0 bis 5°C eine Lösung von 7,6 g (0,11 mol) NaNO₂ in 12 ml H₂O zugetropft. Nach 1 Stunde wird diese Diazoniumsalzsuspension bei 5 bis 10°C zu einer Lösung von 16,3 g (0,1 mol) 2,3-Dichlorphenol und 20 g (0,5 mol) NaOH in 200 ml H₂O zugegeben. Man läßt das Gemisch auf Raumtemperatur erwärmen, rührt 4 Stunden nach, neutralisiert mit wäßriger HCl und saugt den ausgefallenen Feststoff über eine Nutsche ab. Durch Umkristallisieren aus 200 ml Toluol und 20 ml Methanol können 26,3 g (83,5 % der Theorie) des reinen (2,3-Dichlor-4-hydroxyphenyl)-4-sulfonylphenyldiazens gewonnen werden.

### Beispiel 6

In einem 100 ml Zweihalskolben mit Innenthermometer und Magnetrührer wird zu 25 ml wässriger HCI (15 %) und 4,9 g (0,0525 mol) Anilin bei 0 bis 5°C eine Lösung von 3,8 g (0,055 mol) NaNO₂ in 6 ml H₂O zugetropft. Nach 10 Minuten wird diese Diazoniumsalzlösung bei 5 bis 10°C zu einer Lösung von 8,15 g (0,05 mol) 2,3-Dichlorphenol und 10 g (0,25 mol) NaOH in 100 ml H₂O zugegeben. Man läßt das Gemisch auf Raumtemperatur erwärmen, rührt 1 Stunde nach, und neutralisiert mit wässriger HCl (pH = 7). Zu dieser Lösung werden in einem 500 ml Dreihalskolben mit Innenthermometer, Rührer und Gaseinleitung 300 ml Methanol, 2,5 g raney-Nickel und 15 µl Bis(2-Hydroxyethyl)sulfid zugegeben und der Kolben wird argoniert. Bei 40 bis 50°C werden dann in 1,5 Stunden 2300 ml Wasserstoff (0,1 mol) mit ca. 1 bar aufgedrückt. Der Ansatz wird mit etwas Aktivkohle versetzt, weitere 15 Minuten gerührt und über ein Kieselgelpolster filtriert. Nach Abdestilieren des Methanols fällt aus der wässrigen Lösung reines 4-Hydroxy-2,3-dichloranilin mit einer Ausbeute von 83 % an.

### Beispiel 7

In einem 100 ml Zweihalskolben mit Innenthermometer und Magnetrührer wird zu 25 ml wäßriger HCl (15 %) und 4,9 g (0,0525 mol) Anilin bei 0-5°C eine Lösung von 3,8 g (0,055 mol) NaNO₂ in 6 ml H₂O zugetropft. Nach 10 min wird diese Diazoniumsalzlösung bei 5-10°C zu einer Lösung von 8,15 g (0,05 mol) 2,3-Dichlorphenol und 10 g (0,25 mol) NaOH in 100 ml H₂O zugegeben. Man läßt das Gemisch auf Raumtemperatur erwärmen, rührt 1 h nach und neutralisiert mit wäßriger HCl (pH = 7). Zu dieser Lösung werden in einem 500 ml Dreihalskolben mit Innenthermometer, Rührer und Gaseinleitung 300 ml Methanol, 2,5 g Raney-Nickel und 15 µl Bis(2-Hydroxyethyl)sulfid zugegeben und der Kolben wird argoniert. Bei 40-50°C werden dann in 1,5 h 2300 ml Wasserstoff (0,1 mol) mit ca. 1 bar aufgedrückt. Der Ansatz wird mit Aktivkohle versetzt, weitere 15 min gerührt, über ein Kieselgelpolster filtriert und mit wäßriger HCl auf pH = 5,7 gebracht. Nach Abdestillieren des Methanols fallen aus der wäßrigen Lösung 8,4 g (94,4 % d.Th.) reines 4-Hydroxy-2,3-dichloranilin aus.

### Beispiel 8

In einem 250 ml Zweihalskolben mit Innenthermometer und Magnetrührer wird zu 50 ml wäßriger HCl (15 %) und 9,3 g (0,1 mol) Anilin bei 0-5°C eine Lösung von 7,3 g (0,105 mol) NaNO₂ in 22 ml H₂O zugetropft. Nach 30 min wird diese Diazoniumsalzlösung bei 5-10°C zu einer Lösung von 16,4 g (0,1 mol) 2,3-Dichlorphenol und 12,4 g (0,31 mol) NaOH in 100 ml H₂O langsam zugegeben. Man läßt die Suspension auf Raumtemperatur erwärmen, rührt 1 h nach und gibt dann 0,5 g Raney-Nickel zu. Bei 40-50°C werden dann über 2 h 6 g (0,06 mol) Hydrazinhydrat zugegeben. Nach beendeter Zugabe wird 1h nachgerührt und die Lösung wird über ein Kieselgelpolster filtriert. Anschließend wird die Lösung unter Kühlen mit 20 %iger HCI auf pH = 5,7 gebracht. Der ausfallende Feststoff wird mit 50 ml Wasser und 50 ml Toluol gewaschen und getrocknet. Man erhält 16,8 g (94,3 % der Theorie) reines 2,3-Dichlor-4-hydroxyanilin.

### Beispiel 9

In einem 100 ml Zweihalskolben mit Innenthermometer und Magnetrührer wird zu 20 ml wäßriger HCl (15 %) und 3,7 g (0,04 mol) Anilin bei 0-5°C eine Lösung von 2,9 g (0,042 mol) NaNO₂ in 5 ml H₂O zugetropft. Nach 10 min wird diese Diazoniumsalzlösung bei 5-10°C zu einer Lösung von 5,0 g (0,038 mol) 2,3-Difluorphenol und 6,1 g (0,152 mol) NaOH in 60 ml H₂O zugegeben. Man läßt die Lösung auf Raumtemperatur erwärmen, rührt 4 h nach, neutralisiert mit wäßriger HCl und extrahiert 3 x mit je 50 ml Ethylacetat. Die vereinigten organischen Phasen werden mit NaCl-Lösung gewaschen, mit Na₂SO₄ getrocknet und das Lösungsmittel wird im Rotationsverdampfer abdestilliert. Man erhält 8,4 g (94,5 % d.Th.) 2,3-Difluor-4-hydroxyphenyl)phenyldiazen (Schmp. 141°C).

### Beispiel 10

In einem 100 ml Dreihalskolben mit Innenthermometer, Rührer und Gaseinleitung werden 3,5 g (0,015 mol) 2,3-Difluor-4-hydroxyphenyl)phenyldiazen in 30 ml Methanol bei Raumtemperatur gelöst. Der Kolben wird argoniert und anschließend werden 1,5 g Raney-Nickel und 25 µl Bis(2-Hydroxyethyl)sulfid zugegeben. Bei Raumtemperatur werden dann 692 ml Wasserstoff (0,03 mol) mit ca. 1 bar aufgedrückt. Der Ansatz wird mit Aktivkohle versetzt, weitere 15 min gerührt und über ein Kieselgelpolster filtriert. Nach Abdestillieren des Lösungsmittels und Waschen mit n-Hexan erhält man 2,0 g (91,9 % d.Th.) 4-Hydroxy-2,3-difluoranilin.

## Patentansprüche

1. Verfahren zur Herstellung von Verbindungen der Formel (I) 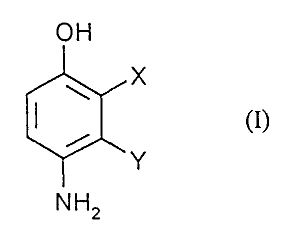 in welcher
X und Y unabhängig voneinander jeweils für Halogen, und CN stehen
durch Umsetzung von
a) Phenolen der Formel (II) 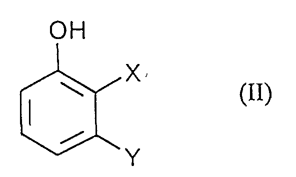 in welcher
X und Y die oben angegebenen Bedeutungen haben,
mit Diazoniumsalzen der Formel (III) 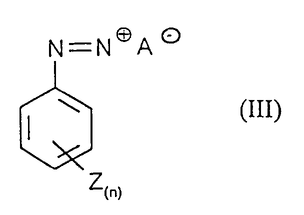 in welcher
n für eine Zahl 0 bis 5,
Z für Halogen, Alkyl, NO₂, COOH, CN und SO₃H steht, wobei Z bei n ≥2 gegebenenfalls unterschiedliche Bedeutungen hat
A⁻ für ein Gegenion steht,
und
b) anschließender Reduktion des nach Verfahrensschritt a) erhältlichen Diazens der Formel (IV) 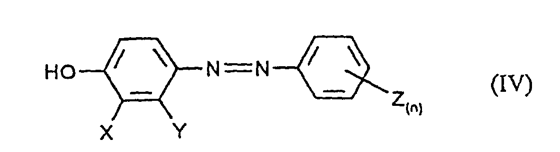 zu der Zielverbindung.

2. Verfahren gemäß Anspruch 1, **dadurch gekennzeichnet, daß** die Umsetzung des Verfahrensschrittes a) in wässrigem Medium bei Temperaturen von 0 bis 50°C durchgeführt wird.

3. Verfahren gemäß Anspruch 1, **dadurch gekennzeichnet, daß** die Reduktion des Verfahrensschrittes b) durch Hydrierung in Gegenwart inerter Lösungsmittel erfolgt.

4. Verfahren gemäß Anspruch 3, **dadurch gekennzeichnet, daß** die Hydrierung mittels Wasserstoff oder Hydrazinhydrat in Gegenwart eines Hilfsmittels bei Temperaturen von 0 bis 150°C durchgeführt wird.

5. Verfahren gemäß Anspruch 1, **dadurch gekennzeichnet, daß** Verbindungen der Formel (II) in denen X und Y unabhängig voneinander für Chlor, Brom, Fluor, und CN stehen, eingesetzt werden.

6. Verfahren gemäß Anspruch 1, **dadurch gekennzeichnet, daß** Verbindungen der Formel (III) in denen n für eine Zahl 0,1, 2 oder 3 und Z für Chlor, Fluor, Brom, NO₂, COOH, CN, SO₃H, Methyl und Ethyl steht und wobei Z bei n ≥2 gegebenenfalls unterschiedliche Bedeutungen hat.

7. Verbindungen der Formel (IV) 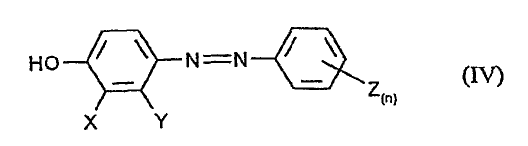 in welcher
X und Y unabhängig voneinander jeweils für Halogen, und CN stehen
n für eine Zahl 0 bis 5 steht
Z für Halogen, Alkyl, NO₂, CN, COOH und SO₃H steht wobei Z bei n ≥2 gegebenenfalls unterschiedliche Bedeutung hat,
ausgenommen ist die Verbindung der Formel (IVa)

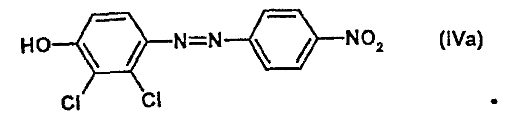

8. Verbindungen gemäß Anspruch 7, in denen
X und Y unabhängig voneinander für Chlor, Brom, Fluor, CN und stehen,
n für eine Zahl 0, 1, 2 oder 3 und
Z für Chlor, Fluor, Brom, CN, COOH, SO₃H, Methyl und Ethyl steht und wobei Z bei n ≥2 gegebenenfalls unterschiedliche Bedeutungen hat,
ausgenommen ist die Verbindung der Formel (IVa)

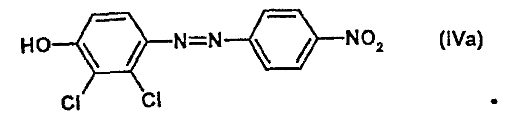

9. Verbindungen gemäß Anspruch 7, in denen
X für Chlor und
Y für Chlor steht,
n für eine Zahl 0, 1, 2 oder 3 und
Z für Chlor, Fluor, Brom, NO₂, CN, COOH, SO₃H, Methyl und Ethyl steht und wobei Z bei n ≥2 gegebenenfalls unterschiedliche Bedeutungen hat,
ausgenommen ist die Verbindung der Formel (IVa)

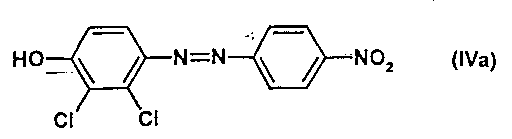

10. Verbindungen gemäß Anspruch 9, in denen n für die Zahl 0 steht.

11. Verbindungen gemäß Anspruch 7, in denen
X und Y für Fluor stehen und
n für eine Zahl 0, 1, 2 oder 3 und
Z für Chlor, Fluor, Brom, NO₂, CN, COOH, SO₃H, Methyl und Ethyl steht und wobei Z bei n ≥ 2 gegebenenfalls unterschiedliche Bedeutungen hat.

12. Verbindungen gemäß Anspruch 11, in denen n für die Zahl 0 steht.

13. Verfahren zur Herstellung von Verbindungen der Formel (IV) 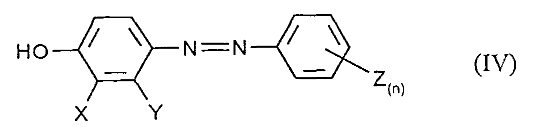 in denen X, Y, Z und n die in Anspruch 7 angegebenen Bedeutungen haben, **dadurch gekennzeichnet, daß** man Phenole der Formel (II) 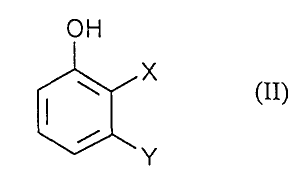 in welcher
X und Y die oben angegebenen Bedeutungen haben, mit Diazoniumsalzen der Formel (III) 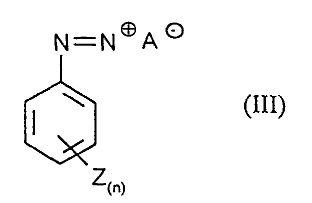 n welcher
n für eine Zahl 0 bis 5,
Z für Halogen, Alkyl, NO₂, COOH, CN und SO₃H steht, wobei Z bei n ≥2 gegebenenfalls unterschiedliche Bedeutungen hat
A⁻ für ein Gegenion steht,
umsetzt.

14. Verwendung der Verbindungen der Formel (IV) als Zwischenprodukte für die Herstellung von Verbindungen der Formel (I) wie in Anspruch 1 definiert.

## Claims

1. Process for preparing compounds of the formula (I) 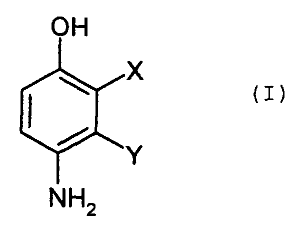 in which
X and Y independently of one another each represent halogen, alkoxy and CN
by reacting
a) phenols of the formula (II) 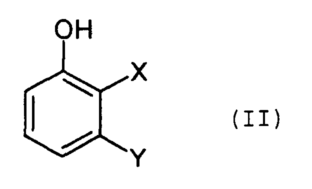 in which
X and Y are as defined above with diazonium salts of the formula (III) 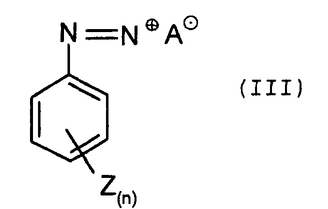 in which
n represents a number 0 to 5,
Z represents halogen, alkyl, NO₂, COOH, CN and SO₃H, Z in the case of n ≥2 optionally having different meanings
A⁻ represents a counterion
and
b) subsequent reduction of the diazene of the formula (IV) 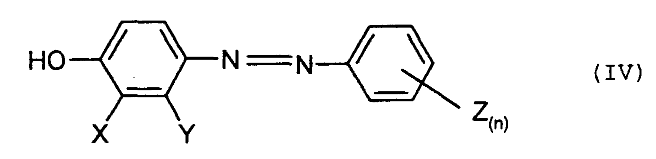 obtainable by process step a) to give the target compound.

2. Process according to Claim 1, **characterized in that** the reaction of process step a) is carried out in an aqueous medium at temperatures of 0 to 50°C.

3. Process according to Claim 1, **characterized in that** the reduction of process step b) is carried out by hydrogenation in the presence of inert solvents.

4. Process according to Claim 3, **characterized in that** the hydrogenation is carried out using hydrogen or hydrazine hydrate in the presence of an auxiliary at temperatures of 0 to 150°C.

5. Process according to Claim 1, **characterized in that** compounds of the formula (II) are used in which X and Y independently of one another each represent chlorine, bromine, fluorine and CN.

6. Process according to Claim 1, **characterized in that** compounds of the formula (III) are used in which n represents a number 0, 1, 2 or 3 and Z represents chlorine, fluorine, bromine, NO₂, COOH, CN, SO₃H, methyl and ethyl, Z in the case of n ≥2 optionally having different meanings.

7. Compounds of the formula (IV) 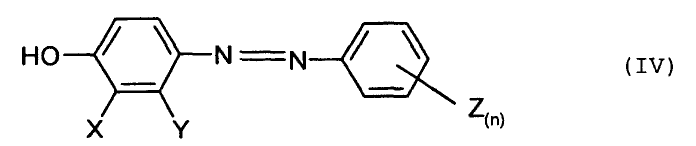 in which
X and Y independently of one another each represent halogen, and CN
n represents a number 0 to 5,
Z represents halogen, alkyl, NO₂, CN, COOH and SO₃H, Z in the case of n ≥2 optionally having different meanings,
with the exception of the compound of the formula (IVa)

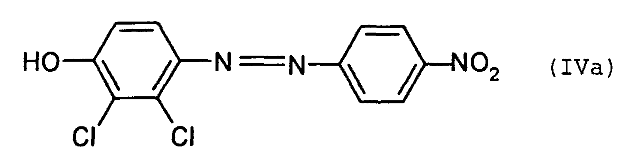

8. Compounds according to Claim 7 in which
X and Y independently of one another each represent chlorine, bromine, fluorine, CN,
n represents a number 0, 1, 2 or 3 and
Z represents chlorine, fluorine, bromine, CN, COOH, SO₃H, methyl and ethyl, Z in the case of n ≥2 optionally having different meanings,
with the exception of the compound of the formula (IVa)

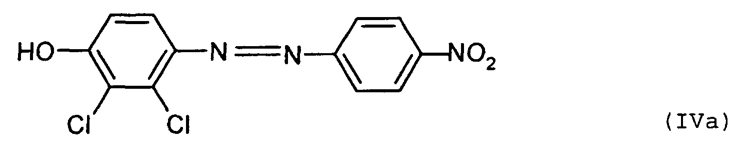

9. Compounds according to Claim 7 in which
X represents chlorine and
Y represents chlorine,
n represents a number 0, 1, 2 or 3 and
Z represents chlorine, fluorine, bromine, NO₂, CN, COOH, SO₃H, methyl and ethyl, Z in the case of n ≥2 optionally having different meanings,
with the exception of the compound of the formula (IVa)

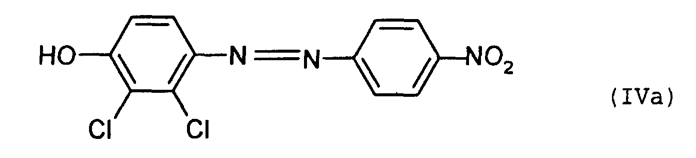

10. Compounds according to Claim 9 in which n represents the number 0.

11. Compounds according to Claim 7 in which
X and Y each represent fluorine and
n represents a number 0, 1, 2 or 3 and
Z represents chlorine, fluorine, bromine, NO₂, CN, COOH, SO₃H, methyl and ethyl,
Z in the case of n ≥2 optionally having different meanings.

12. Compounds according to Claim 11 in which n represents the number 0.

13. Process for preparing compounds of the formula (IV) 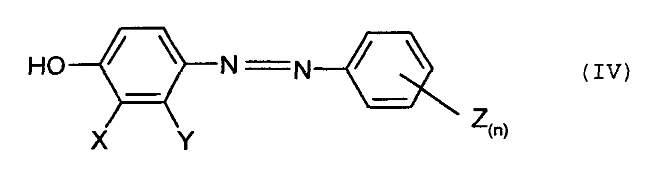 in which X, Y, Z and n are each as defined in Claim 7, **characterized in that** phenols of the formula (II) 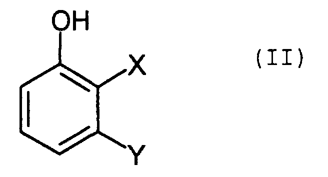 in which
X and Y are each as defined above are reacted with diazonium salts of the formula (III) 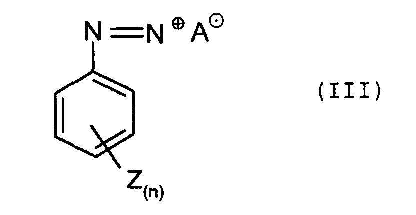 in which
n represents a number 0 to 5,
Z represents halogen, alkyl, NO₂, COON, CN and SO₃H, Z in the case of n ≥2 optionally having different meanings,
A⁻ represents a counterion.

14. The use of compounds of the formula (IV) as intermediates for preparing compounds of the formula (I) as defined in Claim 1.

## Revendications

1. Procédé pour la préparation de composés répondant à la formule (I) 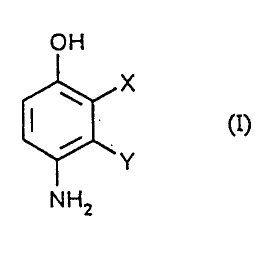 dans laquelle
X et Y représentent, indépendamment l'un de l'autre, respectivement un atome d'halogène et un groupe CN,
par la mise en réaction de
a) phénols répondant à la formule (II) 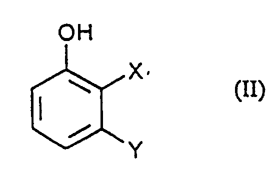 dans laquelle
X et Y ont les significations indiquées ci-dessus,
avec des sels de diazonium répondant à la formule (III) 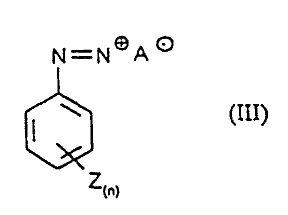 dans laquelle
n représente un nombre de 0 à 5,
Z représente un atome d'halogène, un groupe alkyle, un groupe NO₂, un groupe COOH, un groupe CN et un groupe SO₃H, Z possédant le cas échéant des significations différentes lorsque n ≥ 2,
A⁻ représente un ion complémentaire
et
b) réduction ultérieure du diazène que l'on peut obtenir conformément à l'étape opératoire a) et qui répond à la formule (IV) 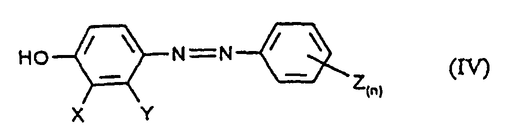 pour obtenir le composé recherché.

2. Procédé selon la revendication 1, **caractérisé en ce qu'**on effectue la mise en réaction de l'étape opératoire a) dans un milieu aqueux à des températures de 0 à 50 °C.

3. Procédé selon la revendication 1, **caractérisé en ce qu'**on effectue la réduction de l'étape opératoire b) par hydrogénation en présence de solvants inertes.

4. Procédé selon la revendication 3, **caractérisé en ce qu'**on effectue l'hydrogénation avec de l'hydrogène ou de l'hydrate d'hydrazine en présence d'un adjuvant à des températures de 0 à 150 °C.

5. Procédé selon la revendication 1, **caractérisé en ce qu'**on met en oeuvre des composés répondant à la formule (II) dans lesquels X et Y représentent, indépendamment l'un de l'autre, un atome de chlore, un atome de brome, un atome de fluor et un groupe CN.

6. Procédé selon la revendication 1, **caractérisé en ce qu'**on met en oeuvre des composés répondant à la formule (III) dans lesquels n représente un nombre égal à 0, 1, 2 ou 3 et Z représente un atome de chlore, un atome de fluor, un atome de brome, un groupe NO₂, un groupe COOH, un groupe CN et un groupe SO₃H, un groupe méthyle et un groupe éthyle', et dans lequel Z possède le cas échéant des significations différentes lorsque n ≥ 2.

7. Composés répondant à la formule (IV) 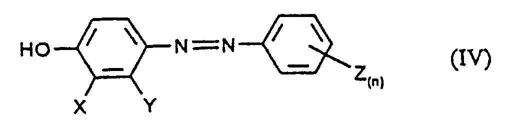 dans laquelle
X et Y représentent, indépendamment l'un de l'autre, respectivement un atome d'halogène et un groupe CN,
n représente un nombre de 0 à 5,
Z représente un atome d'halogène, un groupe alkyle, un groupe NO₂, un groupe CN, un groupe COOH et un groupe SO₃H, Z possédant le cas échéant des significations différentes lorsque n ≥ 2;
à l'exception du composé répondant à la formule (IVa)

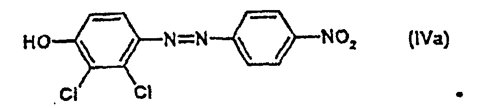

8. Composés selon la revendication 7, dans lesquels
X et Y représentent, indépendamment l'un de l'autre, respectivement un atome de chlore, un atome de brome, un atome de fluor et un groupe CN,
n représente un nombre égale à 0, 1, 2 ou 3,
Z représente un atome de chlore, un atome de fluor, un atome de brome, un groupe CN, un groupe COOH, un groupe SO₃H, un groupe méthyle et un groupe éthyle, Z possédant le cas échéant des significations différentes lorsque n ≥ 2, à l'exception du composé répondant à la formule (IVa)

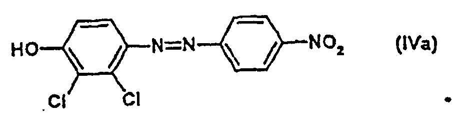

9. composés selon la revendication 7, dans lesquels
X représente un atome de chlore et
Y représente un atome de chlore,
n représente un nombre égal à 0, 1, 2 ou 3, et
Z représente un atome de chlore, un atome de fluor, un atome de brome, un groupe NO₃, un groupe CN, un groupe COOH, un groupe SO₃H, un groupe méthyle et un groupe éthyle, Z possédant le cas échéant des significations différentes lorsque n ≥ 2,
à l'exception du composé répondant à la formule (IVa)

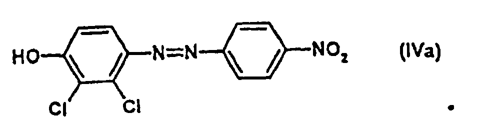

10. Composés selon la revendication 9, dans lesquels n représente le nombre 0.

11. Composés selon la revendication 7, dans lesquels
X et Y représentent un atome de fluor et
n représente un nombre égal à 0, 1, 2 ou 3, et
Z représente un atome de chlore, un atome de fluor, un atome de brome, un groupe NO₃, un groupe CN, un groupe COOH, un groupe SO₃H, un groupe méthyle et un groupe éthyle, Z possédant le cas échéant des significations différentes lorsque n ≥ 2,

12. Composés selon la revendication 11, dans lesquels n représente le nombre 0.

13. Procédé pour la préparation de composé répondant à la formule (IV) 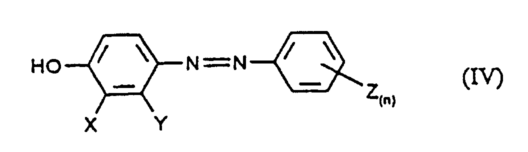 dans laquelle
X, Y, Z et n ont les significations indiquées à la revendication 7, **caractérisé en ce qu'**on fait réagir des phénols répondant à la formule (II) 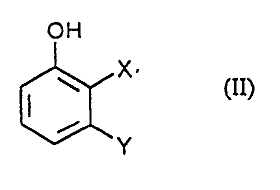 dans laquelle
X et Y ont les significations indiquées ci-dessus, avec des sels de diazonium répondant à la formule (III) 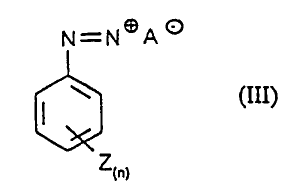 dans laquelle
n représente un nombre de 0 à 5,
Z représente un atome d'halogène, un groupe alkyle, un groupe NO₂, un groupe COOH, un groupe CN et un groupe SO₃H, Z possédant le cas échéant des significations différentes lorsque n ≥ 2,
A⁻ représente un ion complémentaire.

14. Utilisation des composés répondant à la formule (IV) à titre de produits intermédiaires pour la préparation de composés répondant à la formule (I) tels que définis à la revendication 1.
